(19)

Europäisches
Patentamt
European
Patent Office
Office européen
des brevets

(11) **EP 4 609 149 B1**

(12) **EUROPEAN PATENT SPECIFICATION**

(45) Date of publication and mention
of the grant of the patent:
**13.05.2026   Bulletin 2026/20**

(21) Application number: **23800718.1**

(22) Date of filing: **24.10.2023**

(51) International Patent Classification (IPC):
*G01D 18/00* (2006.01)     *G06N 3/0455* (2023.01)
*G06N 3/0464* (2023.01)     *G06N 3/088* (2023.01)

(52) Cooperative Patent Classification (CPC):
**G01D 18/008; G06N 3/0455; G06N 3/0464;
G06N 3/088**

(86) International application number:
**PCT/ZA2023/050066**

(87) International publication number:
**WO 2024/092293 (02.05.2024 Gazette 2024/18)**

(54) **METHOD AND SYSTEM OF CALIBRATION OF A SENSOR OR A NETWORK OF SENSORS**

VERFAHREN UND VORRICHUNG ZUR KALIBRIERUNG EINES SENSORS ODER EINES
SENSORNETZWERKS

MÉTHODE ET SYSTÈME DE CALIBRATION D'UN CAPTEUR OU D'UN RÉSEAU DE CAPTEURS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **25.10.2022   GB 202215800**

(43) Date of publication of application:
**03.09.2025   Bulletin 2025/36**

(73) Proprietor: **University of Cape Town**
**7700 Cape Town (ZA)**

(72) Inventor: **MISHRA, Amit Kumar**
**Cape Town 7700 (ZA)**

(74) Representative: **HGF**
**HGF Limited**
**4th Floor, 1 City Square**
**Leeds LS1 2ES (GB)**

(56) References cited:
**CN-A- 110 730 435**

- **LAURA BALZANO ET AL: "Blind calibration of
sensor networks", INFORMATION PROCESSING
IN SENSOR NETWORKS, 2007. IPSN 2007. 6TH
INTERN ATIONAL SYMPOSIUM ON, IEEE, PI, 25
April 2007 (2007-04-25), pages 79 - 88,
XP058136635, ISBN: 978-1-59593-638-7, DOI:
10.1145/1236360.1236372**
- **WANG YUZHI ET AL: "A Deep Learning Approach
for Blind Drift Calibration of Sensor Networks",
IEEE SENSORS JOURNAL, IEEE, USA, vol. 17,
no. 13, 1 July 2017 (2017-07-01), pages 4158 -
4171, XP011652520, ISSN: 1530-437X, [retrieved
on 20170610], DOI: 10.1109/JSEN.2017.2703885**

## Description

### FIELD OF THE INVENTION

This invention relates to sensor calibration. In particular, the invention relates to calibration of a single sensor or a network of sensors that are in a given environment.

### BACKGROUND TO THE INVENTION

Calibration is a major component of any metrological system especially for sensors which are installed in remote places. Without a thorough investigation and methodology around sensor calibration, the data collected from the sensors are often not reliable. Sensors may be calibrated to an initial high standard; however the quality of the data often quickly deteriorates as the calibration loses accuracy. This is a major pain point in the current day of ubiquitous sensing.

Calibration efforts for individual sensor types are extensive. Most of these processes need a reference sensor or some ground-truths. Running a reference-based calibration for remote sensors is a costly task. Also, it does not scale up; i.e., when the number of sensors is in the hundreds, the task becomes impossible to be carried out on a regular basis.

One of the solutions to this challenge has been to treat the battery of sensors as a single system. This system can, then, be calibrated as a whole rather than focusing on individual sensors in this network. A physics-based model may be used to do this and data from the sensor network is expected to fit the model as closely as possible. Hence, the individual sensor-calibration parameters are fine-tuned to force this fit. Many following works have used this approach and modified it as well. Though efficient, this approach is not fully blind.

Calibration of sensors when a ground-truth or references are not used is referred to as "blind calibration".

Some blind sensor methods require a large number of sensors to work. For example, one method uses an assumption of the existence of spatial oversampling to give an elegant solution which has been leveraged upon by many other works.

Unfortunately, in most real-life cases, the number of sensors available is usually limited.

US10008770B2 discloses calibration of sensors or sensor arrays with beam forming based calibration of antennas. The method leverages on beam forming algorithms and then this is used through a Gaussian source to calibrate the antennas. This is a well-known method of calibration in radar domain.

WO2020191980A1 discloses a blind calibration method for wireless sensor network data drift. This uses a Kalman filter to track the drift in sensor drift. This is not a long-term strategy, because, after a while Kalman-filter will drift itself and will need reference data. Hence, this method cannot correct indefinitely.

The paper of BALZANO L. et Al : "Blind calibration of sensor networks", Information Processing in Sensor Networks, 2007. IPSN 2007. 6th Int. Symp, 25 April 2007, relates to blind calibration of a wireless sensors network.

The preceding discussion of the background to the invention is intended only to facilitate an understanding of the present invention. It should be appreciated that the discussion is not an acknowledgment or admission that any of the material referred to was part of the common general knowledge in the art as at the priority date of the application.

### SUMMARY OF THE INVENTION

According to an aspect of the present invention there is provided a method of calibration of a sensor, wherein the sensor is in a given environment, the method comprising: in a reliable calibration phase after calibration of the sensor, determining an environment response function ($h$) in a first time period based on a known sensor response function ($f$), the determining carried out when obtaining measured values ($y$) by the sensor of a measurand ($x$) of an event ($e$) in the first time period, wherein the environment response function is a spatio-temporal response of factors of the environment representing the environmental response operating on the event to result in the measurand field; in an unreliable calibration phase after the first time period, estimating a current sensor response function ($g$) based on the environment response function ($h$) determined in the reliable calibration phase, the estimating carried out when obtaining measured values ($y$) by the sensor of the measurand field ($x$) of the event ($e$) in a second time period; and outputting a calibration function based on an inverse of the estimated current sensor response function ($g$) for updating calibration of the sensor.

Determining the environment response function ($h$) may determine a value of the function or a shape of the function. Determining the environment response function ($h$) as a shape of the function may be determined from environmental response function results of multiple sensors. The method may include measuring selected factors of the environment over the first time period at the sensor to determine the environmental response function ($h$).

In one embodiment, the method may be a semi-blind calibration using a controlled perturbation in the measurand field of the event to determine the environment response function ($h$) based on the known sensor response function ($f$).

In another embodiment, the method may be a blind calibration including modelling the method as a two stage autoencoder network with each stage implemented by a block of convolutional neural networks, wherein the modelling is trained in the first time period for an event ($e$) and a second time period is selected with closely matching environment factors to the first period to replicate the event ($e$). The two stage autoencoder network may include: a first stage in the form of an observation process block modelling a data dependency from the measured data ($y$) to the measurand field ($x$), wherein the first stage models a calibration function; and a second stage in the form of an environment response block modelling a data dependency from the measurand field ($x$) to the event field ($e$), wherein the second stage models the environment response function.

The method may include training a model using measured data ($y$) from the sensor including: in the reliable calibration phase, training both the first stage and the second stage of the model using the measured data ($y$) and the measurand field ($x$), wherein the measurand field is determined using the known sensor response function ($f$); and in the unreliable calibration phase, the second stage is not trained and the first stage is trained using only the measured data ($y$).

After the unreliable calibration phase, an updated observation process block of the first stage may be used as an updated calibration process.

The method may include adding a representation of measurement noise to the known sensor response function and the current sensor response function.

According to another aspect of the present invention there is provided a method of calibration of a network of sensors, wherein the each of the sensors of the network has a known relative position in the given environment and the method comprises the method of the above method aspect of the present invention.

According to a further aspect of the present invention there is provided computer program stored on a computer readable medium and loadable into the internal memory of a digital computer, comprising software code portions, when said program is run on a computer, for performing the method steps of the above method aspect of the present invention.

According to a further aspect of the present invention there is provided system for calibration of a sensor, wherein the sensor is in a given environment, the system comprising: an environment response determining component for determining an environment response function ($h$) in a first time period in a reliable calibration phase after calibration of the sensor, with the environment response function ($h$) based on a known sensor response function ($f$), the determining carried out when obtaining measured values ($y$) by the sensor of a measurand ($x$) of an event ($e$) in the first time period, wherein the environment response function is a spatio-temporal response of factors of the environment representing the environmental response operating on the event to result in the measurand field; a current sensor response estimating component for estimating in an unreliable calibration phase after the first time period, a current sensor response function based on the environment response function determined in the reliable calibration phase, the estimating carried out when obtaining measured values ($y$) by the sensor of the measurand field ($x$) of the event ($e$) in a second time period after the first time period; and an output component for outputting a calibration function based on an inverse of the estimated current sensor response function for updating calibration of the sensor. The system may calibrate a network of sensors.

The environment response determining component may determine a value of the environment response function or a shape of the environment response function. The environment response determining component may determine the environment response function as a shape of the function and includes a function shape aggregator of environmental response function results of multiple sensors.

The environmental response determining component may include: an environment factor measuring component for measuring selected factors of the environment over the first time period to determine the environmental response function ($h$).

In one embodiment, the system may be a semi-blind calibration system including a controlled perturbation component for applying a controlled perturbation in the measurand field of the event to determine the environment response function ($h$) based on the known sensor response function ($f$).

In another embodiment, the system may be a blind calibration system including a two stage autoencoder network with each stage implemented by a block of convolutional neural networks, wherein the autoencoder network is trained in the first time period for an event ($e$) and a second time period is selected with closely matching environment factors to the first period to replicate the event ($e$). A first stage of the two stage autoencoder network may be an observation process block modelling a data dependency from the measured data ($y$) to the measurand field ($x$), wherein the first stage models a calibration function; and a second stage may be an environment response block modelling a data dependency from the measurand field ($x$) to the event field ($e$), wherein the second stage models the environment response function.

The system may include a training component for training a model using measured data ($y$) from the sensor including: a reliable calibration phase training component for training both the first stage and the second stage of the model using the measured data ($y$) and the measurand field ($x$), wherein the measurand field is determined using the known sensor response function ($f$); and an unreliable calibration phase training component for training the first stage using only the measured data ($y$) and refraining from training the second stage.

After the unreliable calibration phase, an updated observation process block of the first stage may be used as an updated calibration process.

The system may include a noise representing component for adding a representation of measurement noise to the known sensor response function and the current sensor response function.

According to a further aspect of the present invention there is provided a method of training an auto-calibration sensor model for calibration of a sensor, wherein the sensor is in a given environment, the model having: a first stage in the form of an observation process block modelling a data dependency from the measured data ($y$) to the measurand field ($x$) to model a calibration function; and a second stage in the form of an environment response block modelling a data dependency from the measurand field ($x$) to the event field ($e$) to model the environment response function; the method of training comprising: in the reliable calibration phase, training both the first stage and the second stage of the model using the measured data ($y$) and the measurand field ($x$), wherein the measurand field is determined using the known sensor response function ($f$); and in the unreliable calibration phase, the second stage is not trained and the first stage is trained using only the measured data ($y$).

Embodiments of the invention will now be described, by way of example only, with reference to the accompanying drawings.

**BRIEF DESCRIPTION OF THE DRAWINGS**

In the drawings:

| | |
|---|---|
| Figure 1A | is a flow diagram of an example embodiment according to an aspect of the method of the present invention; |
| Figure 1B | is a flow diagram of an example embodiment according to another aspect of the method of the present invention; |
| Figure 2A | is a measurement process flow diagram of an example embodiment of a signal processing system according to an aspect of the present invention; |
| Figure 2B | is a measurement process flow diagram of an example embodiment of a signal processing system including machine learning blocks according to an aspect of the present invention; |
| Figure 2C | is an example embodiment of an autoencoder according to an aspect of the present invention; |
| Figures 3A to 3F | are graphs showing simulation results of an aspect of the present invention; |
| Figure 4 | is a block diagram of an example embodiment of a calibration system in accordance with the present invention; and |
| Figure 5 | illustrates an example of a computing device in which various aspects of the disclosure may be implemented. |

**DETAILED DESCRIPTION WITH REFERENCE TO THE DRAWINGS**

Methods and systems of at least partial autonomous calibration of a sensor or a network of sensors are described where the sensor in a given environment.

There is a need for some invariant feature to help in auto-calibration. In the described approach, the environment's response function at the point of sensing is taken to be an invariant and used for semi-blind or blind calibration of a sensor. The "given environment" is constant or modelled between a sensor and a measurand over time. Solutions are also provided which accommodate any small changes in the environment's response function. By leveraging on the overall system of measurement and modelling this "complete" system, including the response of the environment, automatic calibration is provided.

The method is carried out with a first phase referred to as a "reliable calibration" phase in a first period of time that occurs after calibration of a sensor when the calibration is accurate. Over time the calibration becomes less accurate and a second phase occurs after the first period of time referred to as an "unreliable calibration" phase in which a calibration correction is required to be estimated.

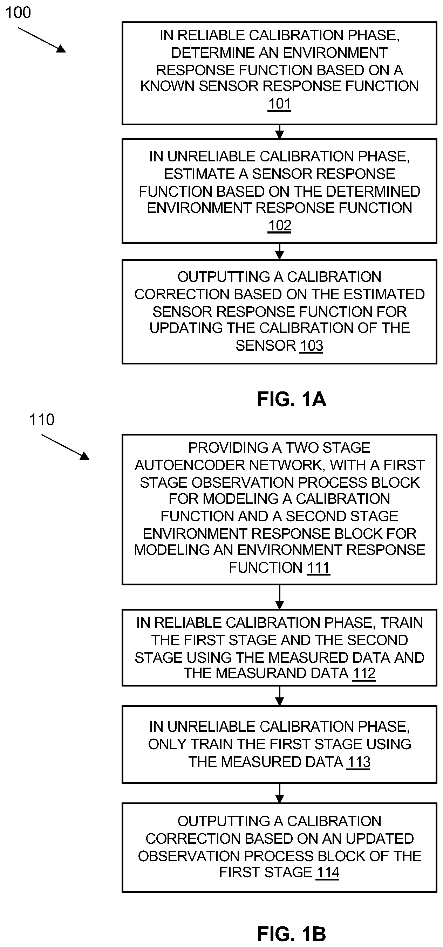

Referring to Figure 1A, a flow diagram (100) shows an example embodiment of the overall method of calibration of a sensor. The method may also be applied to a network of sensors.

An assumption for the described method is that the environment between a sensor and a measurand does not change with time. A sensor may be stationary in the environment such that the environmental factors between the sensor and the measurand do not change over time. A sensor may be moving in a known environment with the change in the environmental factors modelled to accommodate the change. This is referred to as the sensor being in a "given" or "set" environment.

In a reliable calibration phase, in a first time period after calibration of a sensor, the method determines (101) an environment response function ($h$) based on a known sensor response function ($f$). Determining the environmental response function is carried out when obtaining measured values ($y$) by the sensor of a measurand field ($x$) of an event ($e$) in the first time period. The environment response function is a spatio-temporal response of the environment representing the environmental response operating on the event to result in the measurand field. The sensor response function is known as the calibration of the sensor is accurate at this phase.

The first period of time has a duration determined by the period for which the sensor remains accurately calibrated. This first period may be defined based on factors that contribute to a deterioration of the accuracy of the calibration, such as the type of sensor, aging of the sensor, and the environment in which the sensor is being used. The first period may depend on the sensor and the operation or situation. Some sensors may need calibration more frequently than other sensors.

In an unreliable calibration phase, at a time after the first time period, the method estimates (102) a current sensor response function ($g$) based on the environment response function determined in the reliable calibration phase. The current sensor response function ($g$) is an unknown sensor response function due to the unknown effects of the unreliable calibration of the sensor. The estimating is carried out when obtaining measured values ($y$) by the sensor of the measurand field ($x$) of the same event ($e$) in a second time period.

The environmental response function ($h$) is a spatio-temporal response of factors of the environment representing the environmental response to the event ($e$). The factors of the environment will vary depending on the nature of the environment. In a natural world environment, the environmental response for a given area includes multiple environmental factors such as the effects of weather conditions, pollution levels, seasons, daytime, etc. In an industrial environment, the environmental response is more controlled for a given area but may be affected by environmental factors such as the number of people in an area, the number of machines working, etc. An environmental response function is therefore a function of the multiple environmental factors giving the operational conditions of the area and affecting the sensor measurement.

The method requires measurement of selected environment factors at the sensor over the first time period to determine the environmental response function. Even though anything can affect the reading, usually the effect of only a few environmental factors is important and the others are negligible. The environmental factors that are important and that need to be measured depends on the sensor. For example, for a non-dispersive infrared (NDIR) $CO_2$ sensor, humidity is important but temperature is not. Therefore, a fixed set of parameters to be measured to generate the environment response function will depend on an individual sensor.

In one method option, determining the environment response function determines a value of the function. In an alternative method, a feature of the function may be determined such as the shape of the environment response function. In the scenario where a network of sensors is being calibrated, the environment response function as a feature of the function is aggregated from environmental response function results of the multiple sensors in the network.

For the described method, is assumed that the environmental response function ($h$) is invariant and therefore a value may be used. In an alternative approach, a representation of a change in the shape of the environmental response function ($h$) (rather than the exact value of $h$) is used to update the sensor response function in the calibration process. Compared to the exact value of h, the shape of h is less dependent on the effects of variations in the environment.

The method outputs (103) a calibration correction based on the estimated current sensor response function ($g$) with the calibration correction used for updating calibration of the sensor. The sensor response function ($f$) maps the true reading to what the sensor reads. Given the sensor reading, to find the true reading, an inverse of the sensor response function is used. This inverse is called the calibration function. Initially, the calibration function is the inverse "$f^{-1}$" of the sensor response function ($f$) in the reliable calibration phase, and the re-calibration function is the inverse "$g^{-1}$" of the current sensor response function "$g$". A correction to the calibration function may be the inverse of the difference between the sensor response function ($f$) and the current sensor response function ($g$) in the unreliable calibration phase. Once the sensor is re-calibrated, the method may loop to the reliable calibration phase.

The approach is described with two main example embodiments. A first embodiment is partially autonomous and requires some intervention by the user during the calibration process and is therefore referred to as being "semi-blind". In the first embodiment, a semi-blind calibration is provided using a controlled perturbation in the measurand field of the event to determine the environment response function ($h$) based on the known sensor response function ($f$). This embodiment may use conventional numerical methods or machine learning methods. This embodiment requires a human-user to carry out a few steps that can be performed a non-expert.

The environmental response function ($h$) is determined for an event ($e$) in a first time period. In a first embodiment, the event ($e$) may include a controlled perturbation that effects the environmental factors. This provides a semi-blind calibration method. The controlled perturbation of the event ($e$) is then repeated in the form of a later identical controlled perturbation in the second time period. This controlled event may be carried out at any time when recalibration is required. For example, if a sensor is a visual sensor, a controlled perturbation may be the release of a fogging gas in a given location in the region of the sensor with the environmental response measured.

A second embodiment does not require any user intervention and is therefore fully autonomous and is referred to as being "blind". This second embodiment uses machine learning methods to model a calibration system including modelling the method as a two-stage autoencoder network with each stage implemented by a block of convolutional neural networks.

In a second embodiment, the event ($e$) may be a naturally occurring event in a time period at which the environmental response is determined by modelling the environmental response. The second time period may then be selected when conditions of the naturally occurring event are as similar as possible to the first time period. For example, using a similar case as the example of the first embodiment, if a sensor is a visual sensor, the event may be foggy windless weather in a first time period and selection of the second time period is made when similar weather conditions occur.

Determining the environmental response function ($h$) is carried out based on a known sensor response function ($f$) when obtaining measured values ($y$) by the sensor of a measurand field ($x$) of an event ($e$) in the first time period. This may be represented as $y=f(h*e)$, where * represents convolutional function in linear system modeling approaches or a convolutional neural network (CNN) non-linear approach in artificial neural network (ANN) modelling. A CNN has non-linearities in each of the neurons. If the non-linearities are not included, then the CNN will act like a naive convolution. However, when the non-linearities are added, the CNN can be a much more holistic model of the process which is advantageous.

Referring to Figure 1B, a flow diagram (110) shows an example embodiment of the method of the second embodiment of blind calibration of a sensor or a network of sensors.

The method includes providing (111) a two stage autoencoder with a first stage as an observation process block modelling a data dependency from the measured data ($y$) to the measurand field ($x$) to model a calibration function "$f^{-1}$" and a second stage as an environment response block modelling a data dependency from the measurand field ($x$) to the event field ($e$), wherein the second stage models the environment response function.

The method includes, in the reliable calibration phase, training (112) both the first stage and the second stage of the model using the measured data ($y$) and the measurand field ($x$), with the measurand field determined using the known sensor response function ($f$).

The method includes, in the unreliable calibration phase, only training (113) the first stage using only the measured data ($y$). The method outputs (114) in the unreliable calibration phase, an updated observation process block of the first stage for use as an updated calibration process.

MODEL OF THE MEASUREMENT PROCESS

For the sake of generality, it is assumed that there is a single stationary sensor. The treatment can be extended to multiple sensors trivially.

The intention of any measurement system is to measure an event, e, which has created a space-time field. The sensor is used to measure a measurand (true variable of interest), x, at a given location and time. Output from the sensor is a measurement y. The event, e, has been modulated by the spatio-temporal response of the environment to create the measurand field from which x is measured at a given location and time.

**Definition 1:** If time is represented with $t$ and spatial vector by $\vec{s}$, then the spatio-temporal response of the environment is represented by $h(t, \vec{s})$. If it is assumed that the process is linear, then the measurand field, $x(t, \vec{s})$, can be represented as:

$$x(t, \vec{s}) = h(t, \vec{s}) * e(t, \vec{s}) \tag{1}$$

where * represents the convolution operation.

**Definition 2:** The Observation Process (a process that outputs $y$ from $x$) can be modelled by the function $f$ whereas the Restitution Process (of getting the true measurand back from the sensor readings) is modelled by $f^{-1}$. The Observation Process may be as described in M. G. Cox, G. B. Rossi, P. M. Harris, and A. Forbes, "A probabilistic approach to the analysis of measurement processes," Metrologia, vol. 45, no. 5, p. 493, 2008.

Hence, the signal measured by a given sensor at a given time $t$ would be:

$$y(t, \vec{s}) = f\big(x(t, \vec{s})\big) + n(t) = f\big(h(t, \vec{s}) * e(t, \vec{s})\big) + n(t) \tag{2}$$

where $n(t)$ is the measurement noise that cannot be modelled by the processes. The noise may be assumed to be additive white Gaussian noise (AWGN). Hence, it is not made to depend on the location $\vec{s}$. Similarly,

$$\tilde{x}(t, \vec{s}) = f - 1(y(t, \vec{s})) \tag{3}$$

The measurement expression in Equation 1 can be rewritten as:

$$y(t, \vec{s}) = f\big(h(t, \vec{s}) * e(t, \vec{s})\big) + n(t) = f\big(h(t, \vec{s})\big) * f\big(e(t, \vec{s})\big) + n(t) \tag{4}$$

To make the analysis simpler, it is assumed that there is only one sensor and the location parameter is dropped.

$$y(t) = f\big(h(t) * e(t)\big) + n(t) = f\big(h(t)\big) * f\big(e(t)\big) + n(t) \qquad (5)$$

The environment response with one sensor only relies on time, $h(t)$. Where there is more than one sensor, the location information is also embedded in this, $h(s, t)$, where $r$ shows the location.

The environment response $h(s, t)$ is inherently dependent on space and time. Having as many sensors as possible gives the best possible estimation of this. Having a lesser number of sensors is more limiting and a single sensor case is the most limited version.

Having more sensors also adds more known-information to the challenge and, hence, the solution becomes easier to solve.

PROPOSED DESIGN

The variables and functions in this model as discussed in the previous section are listed as follows:

| | |
|---|---|
| $e$: | The event causing the measurand. |
| $h$: | The spatio-temporal response of the environment. |
| $x$: | The measurand field at the sensor. |
| $f$: | The sensor response function of the sensor as known from the observation process. |
| $g$: | The current sensor response function of the sensor estimated in the unreliable calibration phase. |
| $y$: | The measured value of the sensor. |

The life of the sensor in in the field is divided into two phases.

1) Reliable Calibration (RC) Phase: This is when the sensor has been installed in the field recently or recently recalibrated remotely and the calibration model is reliable. In this case, two of the variables/functions from the above list are known, $y$ and f.

2) Unreliable Calibration (UC) Phase: This is when the sensor has been operating in the field after calibration or recalibration for a longer time than the time it takes to create errors and drifts in the calibration function. In this case only $y$ is known reliably.

The purpose of calibration is to make sure that the changes to the sensor response function $f$ that happens over time or due to changes in the operational conditions can be estimated and accommodated.

The described method uses the spatio-temporal response of the environment ($h$) for calibration. The response of the environment, $h$, does not depend on the aging of the sensors. Hence, $h$ can be used as a process-invariant. If $h$ can be estimated in the RC phase (where $f$ is known correctly) then in the UC phase, the known $h$ can be used to estimate the observation process ($g$) of the sensor.

Two embodiments are described to do this.

- Semi-blind Calibration: In this, a controlled perturbation in the event $e$. is used. This can be done by a non-expert and the process would be robust to unpredictable changes in the environment.

- Blind Calibration: To accommodate cases where the above semi-blind calibration process is impossible to carry out, the measurement process may be modeled as a two-stage autoencoder network to provide a fully blind calibration process. An autoencoder is described in I. Goodfellow, Y. Bengio, and A. Courville, Deep learning. MIT press, 2016.

The described method does not require a reference sensor for calibration. This is an advantage. because taking a reference sensor to the site is costly and time consuming. Furthermore, many sites are inaccessible .

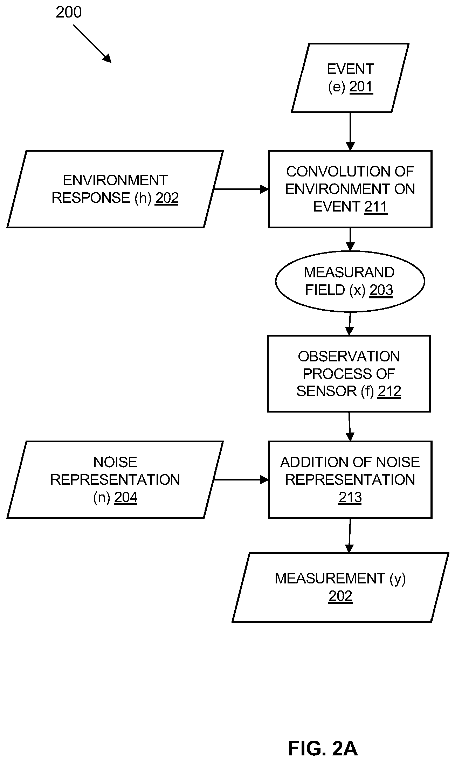

Referring to Figure 2A, a signal flow graph of a measurement process chain (200) in a sensor system is shown using the conventional signal processing blocks (as described in Equation 4):

$$y(t, \vec{s}) = f\big(h(t, \vec{s}) * e(t, \vec{s})\big) + n(t) = f\big(h(t, \vec{s})\big) * f\big(e(t, \vec{s})\big) + n(t) \qquad (4)$$

An event ($e$) (201) and an environment response ($h$) (202) are combined by a convolution operation (211) at a signal processing block to result in a measurand field ($x$) (203) according to Definition 1. The convolution operation (211) may process events (*e*) resulting from the controlled perturbation. The observation process (*f*) (212) of the sensor is provided that operates on the measurand field (*x*) (203) with the addition of a noise representation (213) that is input (*n*) (204), for example, AWGN. This results in an output of measurement (*y*) data (205).

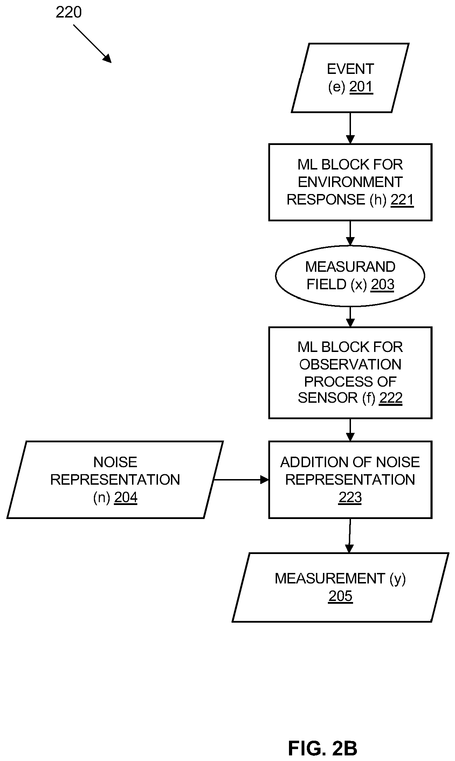

Referring to Figure 2B, a measurement process chain (210) in a sensor system is shown replacing some of the blocks with machine learning based processing blocks.

The convolution operation (211) in Figure 2A combining the event (*e*) (201) and an environment response (*h*) (202) is replaced with a machine learning block (221) for modelling the environmental response (*h*). The machine learning block (221) for modelling the environmental response (*h*) may be an artificial neural network (ANN), for example, a convolutional neural network (CNN) suited to modeling the convolution operation. The observation process (*f*) (212) of Figure 2A is replaced with a machine learning block (222) for modelling the observation process of the sensor (*f*). The machine learning block (222) for modelling the observation process of the sensor (*f*) may be an artificial neural network (ANN).

It can be noted here that the use of artificial neural network (ANN) for observation process (and hence in reference-based calibration) is an active field of research as described in K. Yamamoto, T. Togami, N. Yamaguchi, and S. Ninomiya, "Machine learning-based calibration of low-cost air temperature sensors using environmental data," Sensors, vol. 17, no. 6, p. 1290, 2017 and F. Concas, J. Mineraud, E. Lagerspetz, S. Varjonen, X. Liu, K. Puo- lama ki, P. Nurmi, and S. Tarkoma, "Low-cost outdoor air quality monitoring and sensor calibration: A survey and critical analysis," ACM Transactions on Sensor Networks (TOSN), vol. 17, no. 2, pp. 1-44, 2021.

First Embodiment: Semi-blind Calibration

The existence of a way to create a controlled profile of perturbation in the measurand field is assumed. This is usually possible. For example, if considering particulate material (PM) sensors, a unit function-based perturbation of PM in the vicinity can be created.

The calibration in this case is be carried out following two steps.

Step 1: Referring to the measurement system Equation 4. A known perturbation in event, *e*, is created in the measurand field and in the RC phase the sensor response function *f* is known correctly. Hence, in the RC phase all the terms are known except the environmental response function *h* and the noise.

From this, either numerical methods (following the system diagram shown in Figure 2A) or machine learning methods (following the system diagram shown in Figure 2B) may be used to estimate the environmental response function *h*.

Step 2: In the UC phase, the current sensor response function *g* is not known. However, the environmental response function *h* is known from the previous step. Methods may be used to estimate the current sensor response function *g*. From this the deviation in the environmental response function *h*, $\Delta h$ is determined. This can be used either through numerical methods (following the system diagram as given in Figure 2A) or machine learning methods (following the system diagram as given in Figure 2B) to estimate the difference between the known sensor response function *f* and the current sensor response function *g*, with the difference denoted by *(f-g)* , which then can be used to correct the sensor response function *f*. The inverse of the difference in the sensor response function is the calibration function that is required to correct the sensor. This completes the calibration process.

In the above steps, it is assumed that *h* is invariant. This, in fact, is not strictly the case. Therefore, in an alternative approach, a representation of a change in the shape of *h* (rather than the exact value of *h*) is used to update *f* in the calibration steps proposed above.

The change in the shape of *h* depends on the exact shape of *h*. For example, if the shape is like a Sinc function, then the shape may be represented by methods such as "1- peak-to-first-side-lobe ratio" or "main-lobe-width", etc. If the response more complicated, then it may be described, for example, as a summation of a N number of Gaussian functions. In which case the shape may be represented by the ratio of the largest Gaussian to the smallest; width ratio of the Gaussians etc.

Compared to the exact value of *h*, the shape of *h* is less dependent on the effects of variations in the environment.

Second Embodiment: Blind Calibration

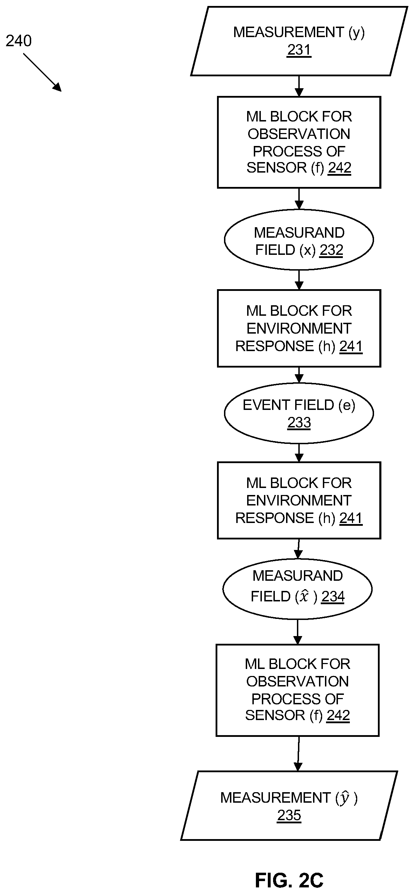

Without the existence of a known perturbation *e* it is impossible to run the above calibration method as there would be more unknowns than known datasets. As we do not have enough information about the measurement process (Equation 4), the structure of the signal flow graph is used to design an autoencoder network (240) with two stages as shown in Figure 2C. The two stage autoencoder models the measurement system. The calibration process usually may be modelled by a polynomial of degree N. For practical purposes, N may be kept less than 3.

The measured data from the sensor, *y*, (231) is used to train this model. The model has two stages (implemented by two blocks of convolutional neural networks (CNNs)). The first stage is an observation process block (242) used to model the data dependency from $y$, the measured data from the sensor, to the measurand field, $x$, (232). The second stage is an environment response block (241) used to model the data dependency from $x$, the measurand field, to the event field, $e$, (233). The autoencoder takes the output of the event field (e) (233) and recreates the measurand field $\bar{x}$ (234) by the environment response block (241) and recreates the measurement field $\bar{y}$ (235) by the observations process block (242).

The autoencoder has four fully connected layers formed of the observation process block (242) and the environment response block (241). The aim is not to compress or find a reduced space representation so the dimensions are kept the same for each layer. The error function is a function of the error between $x$ (232) and $\bar{x}$ (234) as well as between the input measurement $y$ (231) and $\bar{y}$ (235).

Autoencoder-based calibration: The environment response block (241) models the relatively invariant function $h$. Hence, by using a two-stage autoencoder, the calibration function is captured by the observation process block (242).

The calibration in this case shall be carried out following two steps.

Step 1: In the RC phase, the network is trained using both $x$ and $y$ (because $f$ is known correctly). In this step, both the observation process block (242) and environment response block (241) are trained.

Step 2: In the UC phase, the network is trained again. In this case, only $y$ are known, the sensor readings. In this step, the environment response block (241) is not changed. Hence, only the observation process block (242) gets updated. After this step, the updated observation process is used as the updated calibration process.

The above two embodiments may be used either for a single sensor or for a network of sensors. In fact, by having a network of sensors, there is more information about the shape of h. Hence, the calibration process will have improved accuracy.

The methods can be used for any sensors in a given environment. The sensors may be stationary or may have a controlled movement with a modelled change in an environment. One example is in industries. Currently industrial sensors get calibrated every X number of months. These processes are time consuming and costly. Using the described method, the number of months can be increased so that they will need to calibrate less often. Also, full calibration usually means that the industry needs to stop operation for some time when the sensors are getting calibrated. Hence, many industries delay this process. Another advantage is that the methods may be used for sensors in remote locations. A controlled movement of a sensor may be, for example, in an industrial robot.

The following are example scenarios to explain the concept of an environmental response. The environment response is the effect of factors of the environment. For example, there is a forest fire which causes a rise in the temperature that may be a measurand field of a sensor. This rise in temperature is actually infrared waves travelling through the forest. When they travel through the forest, they get affected by the trees and other obstacles, the lie of the land, the wind and other weather conditions, etc. This is the environmental response function (h).

In an industrial setting, there may be an inertial measurement unit to measure the vibration of a robotic arm. The vibration may have been caused at a certain stage in the automation process. Then it gets propagated to the point where the vibration gets measured. This propagation path is the environment response.

Geiger counters may be used as sensors to measure radiation intensities at locations near a nuclear installation. Radiation does not depend on wind speed or temperature. Hence, if there have not been any major construction/demolition/deforestation activities between the installation and the sensor then the environmental response remains the same. Environmental factors may include: weather conditions, pollution conditions, time of day, season, etc. In the first embodiment of the semi-blind method, a controlled perturbation may be used to re-calibrate a sensor. In the blind, autoencoder embodiment, the two time periods (in the known calibration phase and in the unknown calibration phase) are selected when environmental conditions are as close as possible.

There are stations in cities where a municipality measures carbon dioxide levels. Carbon dioxide depends on a range of factors including traffic density, other forms of emissions, etc. Carbon dioxide sensors are also particularly sensitive to humidity. So, if the two time periods (in the known calibration phase and in the unknown calibration phase) are at the same time of the day (such that the traffic density is more or less the same) and it is assumed that the humidity is very close to the level during the first measurement, then the environment response functions would be very similar.

In a factory setup, pyrometers are very useful. They measure temperature. Factory floors are quite controlled environments. Hence, as long as we make sure that the floor is similar (like the number of people on the floor, the state of the machines (e.g. how many assembly lines are working) etc.) in the two cases (first and the later measurements) then the environmental response functions would be similar.

SIMULATION BASED VALIDATION

The autoencoder embodiment may be performed by simulation to illustrate the validation of the model. The different signals and processes of the environment shown in Figure 1B are simulated and modelled

- The event to be measured, e, is modelled as an AWGN.
- The environmental response h is modelled by a 100-tap band-pass filter.

- The calibration function is modelled by a polynomial of degree three,

$$f(x) = x + k_1 x^2 + k_2 x^3$$

- The signal y is fed in batched of 128 data to the autoencoder.
- The autoencoder has four fully connected layers. The aim is not to compress or find a reduced space representation, the dimensions are kept the same for each layer. The error function is a function of the error between x and $\tilde{x}$ as well as between y and $\tilde{y}$, i.e.

$$\alpha L(x, \tilde{x}) + \beta L(y, \tilde{y})$$

$L()$ is the loss function and, in this case, a mean square error is used as the loss function. The parameters $\alpha$ and $\beta$ are used to set the relative importance between the two components of the error function. In this simulation, they are chosen as both to be equal to one. The effect of differential weighting may be applied on the calibration process.

It can be noted here that the coefficients $k_1$ and $k_2$ represent the sensor calibration process and are assumed to drift with aging. For a well calibrated sensor, these two coefficients will be very close to zero and will slowly increase with aging.

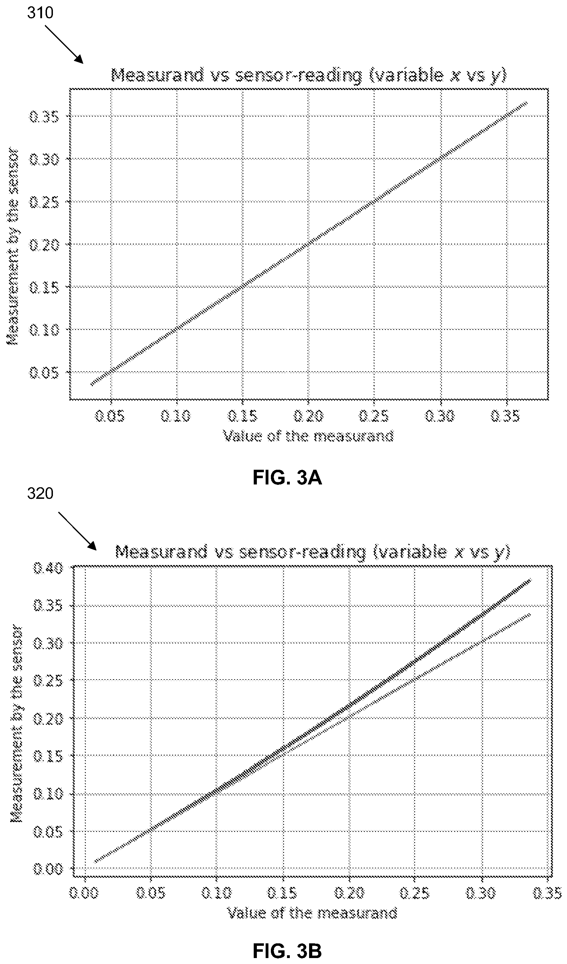

Figures 3A and 3B show graphs (310, 320) of plots of the signal of the measurement by the sensor ($y$) and the measurand ($x$). Figure 3A shows a calibrated sensor where, in the experiments, $k_1 = 0.001$ and $k_2 = 0.0001$. The two plots in this graph (310) are indistinguishable. Figure 3B shows the plots for an uncalibrated situation with $k_1 = 0.3$ and $k_2 = 0.3$ being used. In this graph (320) the two plots diverge.

In the first set of training, the calibrated data is used to train the autoencoder. In the second stage, the higher values of k are used as mentioned above. However, in the second stage, the whole network is not trained; instead, networks between $y$ and $x$, and $\tilde{x}$ and $\tilde{y}$ are trained.

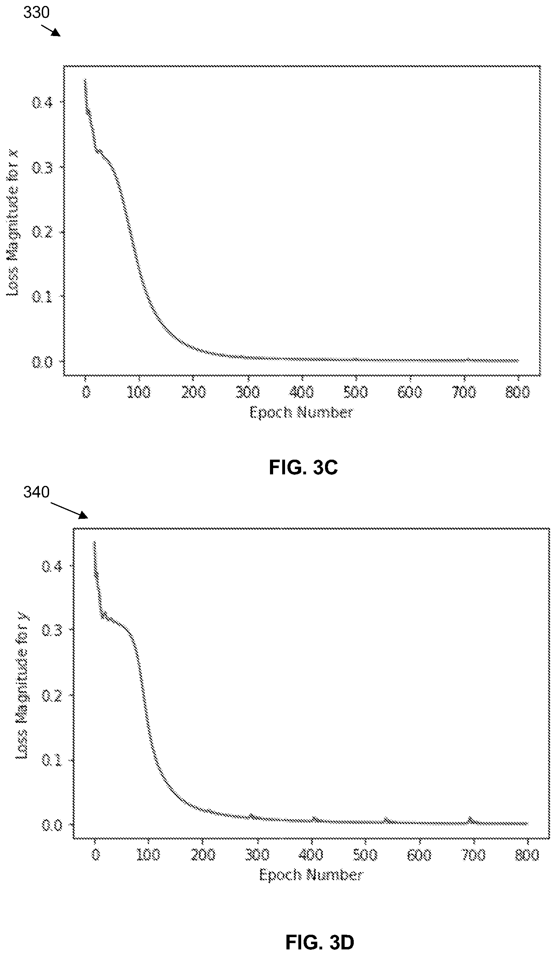

The autoencoder is trained with the initial condition of calibrated sensor ($k_1 = 0.001$ and $k_2 = 0.0001$). Figure 3C shows a graph (330) for the calibrated sensor of loss magnitude for the measurand x against the epoch number of the training. This shows the training process of the autoencoder in terms of learning the value of $x$ the true measurand with a decrease in the loss function shown. Figure 3D shows a graph (340) for the calibrated sensor of the loss magnitude for the measured value $y$ against the epoch number of the training. This shows the training process of the autoencoder in terms of learning the value of $y$ the value measured by the sensor. These figures show a successful training of the autoencoder.

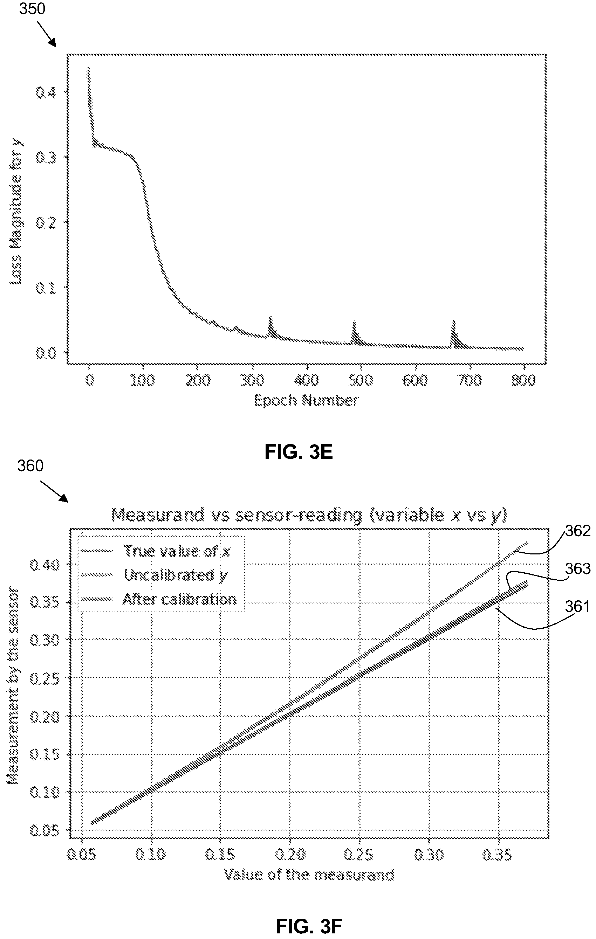

The data with the uncalibrated case ($k_1 = 0.3$ and $k_2 = 0.3$) is then used. In this phase of training only the connections between $x$ and $y$ and $\tilde{x}$ and $\tilde{y}$ are updated. Figure 3E shows a graph (350) for the uncalibrated sensor of the loss magnitude for y against the epoch number of the training. It can be noted that even though the loss function does not decrease as smoothly as the previous cases, it does converge and shows that the training process gets completed successfully.

Next, the trained network (between $y$ and $x$) is used to calibrate the sensor readings. These data are plotted (along with the data from calibrated and uncalibrated cases) in the graph (360) of Figure 3F) of the measurand and measured values $x$ and $y$. Line (361) shows the true value x, line (362) shows an uncalibrated $y$, and line (363) shows $y$ after calibration. It can be seen that the error caused by the drifts in $k_1$ and $k_2$ has been corrected significantly. This validates the use of our proposed calibration method.

Having simulation results showing that the solution works for a single sensor is validating as this is the most challenging situation. If the described calibration correction works in this situation, then it will work in the other situations of multi-sensors and having a very large number of sensors.

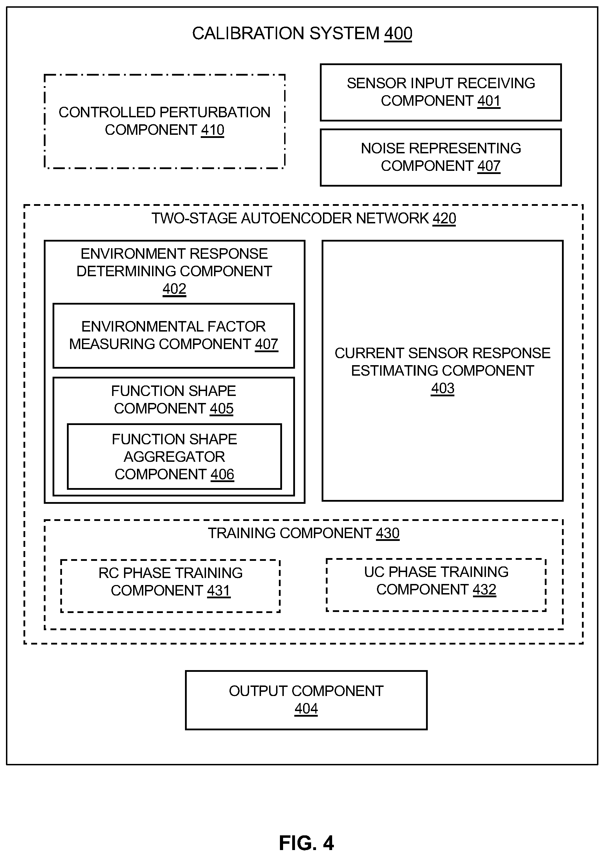

Referring to Figure 4, a block diagram shows an example embodiment of a calibration system (400). The calibration system (400) is illustrated to show both the components of both the first semi-blind embodiment and the blind embodiment. Components that only apply to the blind embodiment are shown in dashed lines and components that only apply to the semi-blind embodiment are shown in dash-dot lines.

The calibration system (400) may be provided on a computing system including a processor for executing the functions of components described below, which may be provided by hardware or by software units executing on the computing system. The software units may be stored in a memory component and instructions may be provided to the processor to carry out the functionality of the described components. In some cases, for example in a cloud computing implementation, software units arranged to manage and/or process data on behalf of the calibration system (400) may be provided remotely.

The calibration system (400) includes a sensor input receiving component (401) for receiving sensor measurements from one or more sensors in their environment. The calibration system (400) includes an environment response determining component (402) for determining an environment response function in a reliable calibration phase in a first time period after calibration of the sensor and a current sensor response estimating component (403) for estimating in an unreliable calibration phase in a second time period after the first time period, a current sensor response function based on the environment response function determined in the reliable calibration phase. The calibration system (400) includes an output component (404) for outputting a calibration function based on the estimated current sensor response function for updating calibration of a sensor.

The environmental response determining component (402) may include an environment factor measuring component (407) for measuring selected factors of the environment over the first time period at the sensor to determine the environmental response function. The environment response determining component (402) may include a function shape component (405) for determining a shape of the environment response function. The function shape component (405) may include a function shape aggregator (406) of environmental response function based on results of multiple sensors.

The calibration system (400) may include a noise representing component (407) for adding a representation of measurement noise to the known sensor response function and the current sensor response function.

In the embodiment of the calibration system (400) as is a semi-blind calibration system, the calibration system (400) may include a controlled perturbation component (410) for controlling or applying a controlled perturbation in the measurand field of the event to determine the environment response function based on the known sensor response function.

In the embodiment of the calibration system (400) as a blind calibration system, a two stage autoencoder network (420) with each stage implemented by a block of convolutional neural networks provides the functionality of the environment response determining component (402) and the current sensor response estimating component (403)

The two stage autoencoder network (420) includes: a first stage as an observation process block modelling a data dependency from the measured data to the measurand field to model a calibration function; and a second stage as an environment response block modelling a data dependency from the measurand field to the event field to model the environment response function.

In the blind calibration system embodiment, the calibration system (400) may include a training component (430) for training a model using measured data from the sensor including: a reliable calibration phase training component (431) for training both the first stage and the second stage of the model using the measured data and the measurand field, with the measurand field is determined using the known sensor response function; and an unreliable calibration phase training component (432) for training the first stage using only the measured data and refraining from training the second stage.

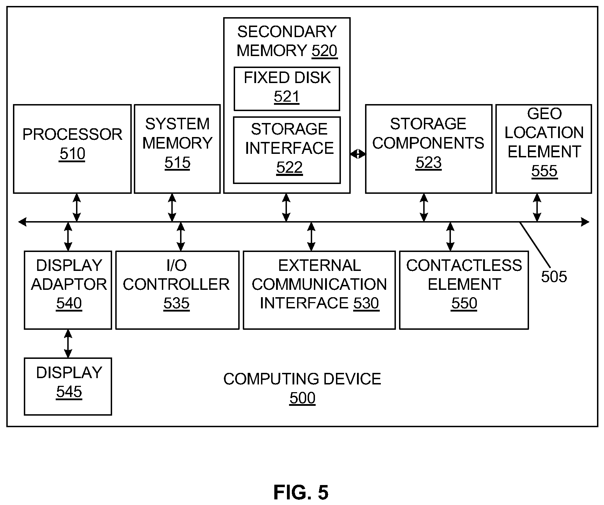

Figure 5 illustrates an example of a computing device (500) in which various aspects of the disclosure may be implemented. For example, the computing device (500) may be used to execute the autoencoder of Figure 3C. The computing device (500) may be embodied as any form of data processing device including a personal computing device (e.g. laptop or desktop computer), a server computer (which may be self-contained, physically distributed over a number of locations), a client computer, or a communication device, such as a mobile phone (e.g. cellular telephone), satellite phone, tablet computer, personal digital assistant or the like. Different embodiments of the computing device may dictate the inclusion or exclusion of various components or subsystems described below.

The computing device (500) may be suitable for storing and executing computer program code. The various participants and elements in the previously described system diagrams may use any suitable number of subsystems or components of the computing device (500) to facilitate the functions described herein. The computing device (500) may include subsystems or components interconnected via a communication infrastructure (505) (for example, a communications bus, a network, etc.). The computing device (500) may include one or more processors (510) and at least one memory component in the form of computer-readable media. The one or more processors (510) may include one or more of: CPUs, graphical processing units (GPUs), microprocessors, field programmable gate arrays (FPGAs), application specific integrated circuits (ASICs) and the like. In some configurations, a number of processors may be provided and may be arranged to carry out calculations simultaneously. In some implementations various subsystems or components of the computing device (500) may be distributed over a number of physical locations (e.g. in a distributed, cluster or cloud-based computing configuration) and appropriate software units may be arranged to manage and/or process data on behalf of remote devices.

The memory components may include system memory (515), which may include read only memory (ROM) and random access memory (RAM). A basic input/output system (BIOS) may be stored in ROM. System software may be stored in the system memory (515) including operating system software. The memory components may also include secondary memory (520). The secondary memory (520) may include a fixed disk (521), such as a hard disk drive, and, optionally, one or more storage interfaces (522) for interfacing with storage components (523), such as removable storage components (e.g. magnetic tape, optical disk, flash memory drive, external hard drive, removable memory chip, etc.), network attached storage components (e.g. NAS drives), remote storage components (e.g. cloud-based storage) or the like.

The computing device (500) may include an external communications interface (530) for operation of the computing device (500) in a networked environment enabling transfer of data between multiple computing devices (500) and/or the Internet. Data transferred via the external communications interface (530) may be in the form of signals, which may be electronic, electromagnetic, optical, radio, or other types of signal. The external communications interface (530) may enable communication of data between the computing device (500) and other computing devices including servers and external storage facilities. Web services may be accessible by and/or from the computing device (500) via the communications interface (530).

The external communications interface (530) may be configured for connection to wireless communication channels (e.g., a cellular telephone network, wireless local area network (e.g. using Wi-Fi™), satellite-phone network, Satellite Internet Network, etc.) and may include an associated wireless transfer element, such as an antenna and associated circuitry.

The computer-readable media in the form of the various memory components may provide storage of computer-executable instructions, data structures, program modules, software units and other data. A computer program product may be provided by a computer-readable medium having stored computer-readable program code executable by the central processor (510). A computer program product may be provided by a non-transient or non-transitory computer-readable medium, or may be provided via a signal or other transient or transitory means via the communications interface (530).

Interconnection via the communication infrastructure (505) allows the one or more processors (510) to communicate with each subsystem or component and to control the execution of instructions from the memory components, as well as the exchange of information between subsystems or components. Peripherals (such as printers, scanners, cameras, or the like) and input/output (I/O) devices (such as a mouse, touchpad, keyboard, microphone, touch-sensitive display, input buttons, speakers and the like) may couple to or be integrally formed with the computing device (500) either directly or via an I/O controller (535). One or more displays (545) (which may be touch-sensitive displays) may be coupled to or integrally formed with the computing device (500) via a display or video adapter (540).

The foregoing description has been presented for the purpose of illustration; it is not intended to be exhaustive or to limit the invention to the precise forms disclosed. Persons skilled in the relevant art can appreciate that many modifications and variations are possible in light of the above disclosure.

Any of the steps, operations, components or processes described herein may be performed or implemented with one or more hardware or software units, alone or in combination with other devices. Components or devices configured or arranged to perform described functions or operations may be so arranged or configured through computer-implemented instructions which implement or carry out the described functions, algorithms, or methods. The computer-implemented instructions may be provided by hardware or software units. In one embodiment, a software unit is implemented with a computer program product comprising a non-transient or non-transitory computer-readable medium containing computer program code, which can be executed by a processor for performing any or all of the steps, operations, or processes described. Software units or functions described in this application may be implemented as computer program code using any suitable computer language such as, for example, Java™, C++, or Perl™ using, for example, conventional or object-oriented techniques. The computer program code may be stored as a series of instructions, or commands on a non-transitory computer-readable medium, such as a random access memory (RAM), a read-only memory (ROM), a magnetic medium such as a hard-drive, or an optical medium such as a CD-ROM. Any such computer-readable medium may also reside on or within a single computational apparatus, and may be present on or within different computational apparatuses within a system or network.

Flowchart illustrations and block diagrams of methods, systems, and computer program products according to embodiments are used herein. Each block of the flowchart illustrations and/or block diagrams, and combinations of blocks in the flowchart illustrations and/or block diagrams, may provide functions which may be implemented by computer readable program instructions. In some alternative implementations, the functions identified by the blocks may take place in a different order to that shown in the flowchart illustrations.

Some portions of this description describe the embodiments of the invention in terms of algorithms and symbolic representations of operations on information. These algorithmic descriptions and representations, such as accompanying flow diagrams, are commonly used by those skilled in the data processing arts to convey the substance of their work effectively to others skilled in the art. These operations, while described functionally, computationally, or logically, are understood to be implemented by computer programs or equivalent electrical circuits, microcode, or the like. The described operations may be embodied in software, firmware, hardware, or any combinations thereof.

The language used in the specification has been principally selected for readability and instructional purposes, and it may not have been selected to delineate or circumscribe the inventive subject matter. It is therefore intended that the scope of the invention be limited not by this detailed description, but rather by the appended claims.

Finally, throughout the specification and any accompanying claims, unless the context requires otherwise, the word 'comprise' or variations such as 'comprises' or 'comprising' will be understood to imply the inclusion of a stated integer or group of integers but not the exclusion of any other integer or group of integers.

**Claims**

1. A method of calibration of a sensor, wherein the sensor is in a given environment, the method comprising:

   in a reliable calibration phase after calibration of the sensor, determining (101) an environment response function (h) in a first time period based on a known sensor response function (f), the determining carried out when obtaining measured values (y) by the sensor of a measurand field (x) of an event (e) in the first time period, wherein the environment response function is a spatio-temporal response of factors of the environment representing the environmental response operating on the event to result in the measurand field;
   in an unreliable calibration phase after the first time period, estimating (102) a current sensor response function (g) based on the environment response function (h) determined in the reliable calibration phase, the estimating carried out when obtaining measured values (y) by the sensor of the measurand field (x) of the event (e) in a second time period; and
   outputting (103) a calibration function based on an inverse of the estimated current sensor response function (g) for updating calibration of the sensor.

2. The method of claim 1, wherein determining the environment response function (h) determines one of the group of: a value of the function, a shape of the function determined from results of one sensor; a shape of the function determined from environmental response function results of multiple sensors.

3. The method of claim 1 or claim 2, including:
   measuring selected factors of the environment over the first time period at the sensor to determine the environmental response function (h).

4. The method of any one of claims 1 to 3, wherein the method is a semi-blind calibration using a controlled perturbation in the measurand field (x) of the event to determine the environment response function (h) based on the known sensor response function (f).

5. The method of any one of claims 1 to 3, wherein the method is a blind calibration including modelling (111) the method as a two stage autoencoder network with each stage implemented by a block of convolutional neural networks, wherein the modelling is trained in the first time period for an event (e) and a second time period is selected with closely matching environment factors to the first period to replicate the event (e).

6. The method of claim 5, wherein:

   a first stage is an observation process block (242) modelling a data dependency from the measured data (y) to the measurand field (x), wherein the first stage models a calibration function; and
   a second stage is an environment response block (241) modelling a data dependency from the measurand field (x) to the event field (e), wherein the second stage models the environment response function.

7. The method of claim 5 or claim 6, including training a model using measured data (y) from the sensor including:

   in the reliable calibration phase, training (112) both the first stage and the second stage of the model using the measured data (y) and the measurand field (x), wherein the measurand field is determined using the known sensor response function (f); and
   in the unreliable calibration phase, the second stage is not trained and the first stage is trained (113) using only the measured data (y).

8. The method of any one of claims 5 to 7, wherein, after the unreliable calibration phase, an updated observation process block of the first stage is used as an updated calibration process.

9. The method of any one of the preceding claims, including adding (213, 223) a representation (204) of measurement noise to the known sensor response function and the current sensor response function.

10. A method of calibration of a network of sensors, wherein the each of the sensors of the network has a known relative position in the given environment, the method comprising the method of any one of claims 1 to 9 applied to each of the sensors.

11. A system for calibration of a sensor, wherein the sensor is in a given environment, the system comprising:

an environment response determining component (402) for determining an environment response function (h) in a first time period in a reliable calibration phase after calibration of the sensor, with the environment response function (h) based on a known sensor response function (f), the determining carried out when obtaining measured values (y) by the sensor of a measurand (x) of an event (e) in the first time period, wherein the environment response function is a spatio-temporal response of factors of the environment representing the environmental response operating on the event to result in the measurand field;

a current sensor response estimating component (403) for estimating in an unreliable calibration phase after the first time period, a current sensor response function based on the environment response function (h) determined in the reliable calibration phase, the estimating carried out when obtaining measured values (y) by the sensor of the measurand field (x) of the event (e) in a second time period after the first time period; and

an output component (404) for outputting a calibration function based on an inverse of the estimated current sensor response function for updating calibration of the sensor.

12. The system of claim 11, wherein the environmental response determining component (402) includes:
an environment factor measuring component (407) for measuring selected factors of the environment over the first time period at the sensor to determine the environmental response function (h).

13. The system of any one of claims 11 to 12, wherein the system is a blind calibration system including a two stage autoencoder network (430) with each stage implemented by a block of convolutional neural networks, wherein the autoencoder network is trained in the first time period for an event (e) and a second time period is selected with closely matching environment factors to the first period to replicate the event (e).

14. The system of claim 13, including a training component (430) for training a model using measured data (y) from the sensor including:

a reliable calibration phase training component (431) for training both the first stage and the second stage of the model using the measured data (y) and the measurand field (x), wherein the measurand field is determined using the known sensor response function (f); and

an unreliable calibration phase training component (432) for training the first stage using only the measured data (y) and refraining from training the second stage.

15. A computer program stored on a computer readable medium and loadable into an internal memory of a digital computer, comprising software code portions, when said program is run on a computer, for performing the method steps of any of the claims 1 to 10.

**Patentansprüche**

1. Verfahren zur Kalibrierung eines Sensors, wobei sich der Sensor in einer gegebenen Umgebung befindet, wobei das Verfahren Folgendes umfasst:

in einer zuverlässigen Kalibrierungsphase nach Kalibrierung des Sensors, Bestimmen (101) einer Umgebungsreaktionsfunktion (h) in einer ersten Zeitperiode basierend auf einer bekannten Sensorreaktionsfunktion (f), wobei das Bestimmen beim Erhalten gemessener Werte (y) durch den Sensor eines Messgrößenfelds (x) eines Ereignisses (e) in der ersten Zeitperiode ausgeführt wird, wobei die Umgebungsreaktionsfunktion eine räumlich-zeitliche Reaktion von Faktoren der Umgebung ist, die die auf das Ereignis wirkende Umgebungsreaktion, mit dem Messgrößenfeld als Ergebnis, darstellen;

in einer unzuverlässigen Kalibrierungsphase nach der ersten Zeitperiode, Schätzen (102) einer Stromsensorreaktionsfunktion (g) basierend auf der in der zuverlässigen Kalibrierungsphase bestimmten Umgebungsreaktionsfunktion (h), wobei das Schätzen beim Erhalten gemessener Werte (y) durch den Sensor des Messgrößenfelds (x) des Ereignisses (e) in einer zweiten Zeitperiode ausgeführt wird; und

Ausgeben (103) einer Kalibrierungsfunktion basierend auf einem Inversen der geschätzten Stromsensorreaktionsfunktion (g) zum Aktualisieren von Kalibrierung des Sensors.

2. Verfahren nach Anspruch 1, wobei das Bestimmen der Umgebungsreaktionsfunktion (h) eines aus der Gruppe von Folgendem bestimmt: einem Wert der Funktion, einer Form der Funktion, die aus Ergebnissen eines Sensors bestimmt wird; einer Form der Funktion, die aus Umgebungsreaktionsfunktionsergebnissen multipler Sensoren bestimmt wird.

3. Verfahren nach Anspruch 1 oder Anspruch 2, beinhaltend:
Messen ausgewählter Faktoren der Umgebung über die erste Zeitperiode an dem Sensor, um die Umgebungsreaktionsfunktion (h) zu bestimmen.

4. Verfahren nach einem der Ansprüche 1 bis 3, wobei das Verfahren eine halbblinde Kalibrierung unter Verwendung einer gesteuerten Störung in dem Messgrößenfeld (x) des Ereignisses ist, um die Umgebungsreaktionsfunktion (h) basierend auf der bekannten Sensorreaktionsfunktion (f) zu bestimmen.

5. Verfahren nach einem der Ansprüche 1 bis 3, wobei das Verfahren eine blinde Kalibrierung ist, die ein Modellieren (111) des Verfahrens als ein zweistufiges Autoencoder-Netzwerk beinhaltet, wobei jede Stufe durch einen Block von faltungsneuronalen Netzwerken implementiert wird, wobei das Modellieren in der ersten Zeitperiode für ein Ereignis (e) trainiert wird und eine zweite Zeitperiode mit Umgebungsfaktoren, die eng mit der ersten Periode übereinstimmen, ausgewählt wird, um das Ereignis (e) zu replizieren.

6. Verfahren nach Anspruch 5, wobei:

eine erste Stufe ein Beobachtungsprozessblock (242) ist, der eine Datenabhängigkeit von den gemessenen Daten (y) zu dem Messgrößenfeld (x) modelliert, wobei die erste Stufe eine Kalibrierungsfunktion modelliert; und
eine zweite Stufe ein Umgebungsreaktionsblock (241) ist, der eine Datenabhängigkeit von dem Messgrößenfeld (x) zu dem Ereignisfeld (e) modelliert, wobei die zweite Stufe die Umgebungsreaktionsfunktion modelliert.

7. Verfahren nach Anspruch 5 oder Anspruch 6, beinhaltend das Trainieren eines Modells unter Verwendung gemessener Daten (y) aus dem Sensor, beinhaltend:

in der zuverlässigen Kalibrierungsphase, Trainieren (112) sowohl der ersten Stufe als auch der zweiten Stufe des Modells unter Verwendung der gemessenen Daten (y) und des Messgrößenfelds (x), wobei das Messgrößenfeld unter Verwendung der bekannten Sensorreaktionsfunktion (f) bestimmt wird; und
wobei in der unzuverlässigen Kalibrierungsphase die zweite Stufe nicht trainiert wird und die erste Stufe nur unter Verwendung der gemessenen Daten (y) trainiert wird (113).

8. Verfahren nach einem der Ansprüche 5 bis 7, wobei, nach der unzuverlässigen Kalibrierungsphase, ein aktualisierter Beobachtungsprozessblock der ersten Stufe als ein aktualisierter Kalibrierungsprozess verwendet wird.

9. Verfahren nach einem der vorhergehenden Ansprüche, beinhaltend das Hinzufügen (213, 223) einer Darstellung (204) von Messrauschen zu der bekannten Sensorreaktionsfunktion und der Stromsensorreaktionsfunktion.

10. Verfahren zur Kalibrierung eines Sensornetzwerks, wobei jeder der Sensoren des Netzwerks eine bekannte relative Position in der gegebenen Umgebung aufweist, wobei das Verfahren das Verfahren nach einem der Ansprüche 1 bis 9, das auf jeden der Sensoren angewendet wird, umfasst.

11. System zur Kalibrierung eines Sensors, wobei sich der Sensor in einer gegebenen Umgebung befindet, wobei das System Folgendes umfasst:

eine Umgebungsreaktionsbestimmungskomponente (402) zum Bestimmen einer Umgebungsreaktionsfunktion (h) in einer ersten Zeitperiode in einer zuverlässigen Kalibrierungsphase nach Kalibrierung des Sensors, wobei die Umgebungsreaktionsfunktion (h) auf einer bekannten Sensorreaktionsfunktion (f) basiert, wobei das Bestimmen beim Erhalten gemessener Werte (y) durch den Sensor einer Messgröße (x) eines Ereignisses (e) in der ersten Zeitperiode ausgeführt wird, wobei die Umgebungsreaktionsfunktion eine räumlich-zeitliche Reaktion von Faktoren der Umgebung ist, die die auf das Ereignis wirkende Umgebungsreaktion, mit dem Messgrößenfeld als Ergebnis, darstellen;
eine Stromsensorreaktionsschätzungskomponente (403) zum Schätzen, in einer unzuverlässigen Kalibrierungsphase nach dem ersten Zeitraum, einer Stromsensorreaktionsfunktion basierend auf der in der zuverlässigen Kalibrierungsphase bestimmten Umgebungsreaktionsfunktion (h), wobei das Schätzen beim Erhalten gemessener Werte (y) durch den Sensor des Messgrößenfelds (x) des Ereignisses (e) in einer zweiten Zeitperiode nach der ersten Zeitperiode ausgeführt wird; und

eine Ausgabekomponente (404) zum Ausgeben einer Kalibrierungsfunktion basierend auf einem Inversen der geschätzten Stromsensorreaktionsfunktion zum Aktualisieren von Kalibrierung des Sensors.

12. System nach Anspruch 11, wobei die Umgebungsreaktionsbestimmungskomponente (402) Folgendes beinhaltet: eine Umgebungsfaktormesskomponente (407) zum Messen ausgewählter Faktoren der Umgebung über die erste Zeitperiode an dem Sensor, um die Umgebungsreaktionsfunktion (h) zu bestimmen.

13. System nach einem der Ansprüche 11 bis 12, wobei das System ein blindes Kalibrierungssystem ist, das ein zweistufiges Autoencoder-Netzwerk (430) beinhaltet, wobei jede Stufe durch einen Block von faltungsneuronalen Netzwerken implementiert wird, wobei das Autoencoder-Netzwerk in der ersten Zeitperiode für ein Ereignis (e) trainiert wird und eine zweite Zeitperiode mit Umgebungsfaktoren, die eng mit der ersten Periode übereinstimmen, ausgewählt wird, um das Ereignis (e) zu replizieren.

14. System nach Anspruch 13, beinhaltend eine Trainingskomponente (430) zum Trainieren eines Modells unter Verwendung gemessener Daten (y) aus dem Sensor, beinhaltend:

eine zuverlässige Kalibrierungsphasentrainingskomponente (431) zum Trainieren sowohl der ersten Stufe als auch der zweiten Stufe des Modells unter Verwendung der gemessenen Daten (y) und des Messgrößenfelds (x), wobei das Messgrößenfeld unter Verwendung der bekannten Sensorreaktionsfunktion (f) bestimmt wird; und eine unzuverlässige Kalibrierungsphasentrainingskomponente (432) zum Trainieren der ersten Stufe nur unter Verwendung der gemessenen Daten (y) und Unterlassen des Trainings der zweiten Stufe.

15. Computerprogramm, das auf einem computerlesbaren Medium gespeichert ist und in einen internen Speicher eines digitalen Computers geladen werden kann, umfassend Softwarecodeabschnitte, wenn das Programm auf einem Computer läuft, zum Durchführen der Verfahrensschritte nach einem der Ansprüche 1 bis 10.

**Revendications**

1. Procédé de calibration d'un capteur, dans lequel le capteur est dans un environnement donné, le procédé comprenant :

dans une phase de calibration fiable après la calibration du capteur, la détermination (101) d'une fonction de réponse d'environnement (h) dans une première période temporelle sur la base d'une fonction de réponse de capteur connue (f), la détermination étant effectuée lors de l'obtention de valeurs mesurées (y) par le capteur d'un champ de mesurande (x) d'un événement (e) dans la première période temporelle, dans lequel la fonction de réponse d'environnement est une réponse spatio-temporelle de facteurs de l'environnement représentant la réponse environnementale opérant sur l'événement pour aboutir au champ de mesurande ; dans une phase de calibration non fiable après la première période temporelle, l'estimation (102) d'une fonction de réponse de capteur actuelle (g) sur la base de la fonction de réponse d'environnement (h) déterminée dans la phase de calibration fiable, l'estimation étant effectuée lors de l'obtention de valeurs mesurées (y) par le capteur du champ de mesurande (x) de l'événement (e) dans une seconde période temporelle ; et l'émission en sortie (103) d'une fonction de calibration sur la base d'un inverse de la fonction de réponse de capteur actuelle estimée (g) en vue de mettre à jour la calibration du capteur.

2. Procédé de la revendication 1, dans lequel la détermination de la fonction de réponse d'environnement (h) détermine l'un du groupe de : une valeur de la fonction, une forme de la fonction déterminée à partir de résultats d'un capteur ; une forme de la fonction déterminée à partir de résultats de la fonction de réponse environnementale de plusieurs capteurs.

3. Procédé de la revendication 1 ou de la revendication 2, comprenant : la mesure de facteurs sélectionnés de l'environnement au cours de la première période temporelle au niveau du capteur pour déterminer la fonction de réponse environnementale (h).

4. Procédé de l'une quelconque des revendications 1 à 3, dans lequel le procédé est une calibration semi-aveugle à l'aide d'une perturbation régulée dans le champ de mesurande (x) de l'événement pour déterminer la fonction de réponse d'environnement (h) sur la base de la fonction de réponse de capteur connue (f).

**5.** Procédé de l'une quelconque des revendications 1 à 3, dans lequel le procédé est une calibration aveugle comprenant la modélisation (111) du procédé en tant que réseau d'autocodeur à deux stades, chaque stade étant mis en œuvre par un bloc de réseaux neuronaux convolutifs, dans lequel la modélisation est entraînée dans la première période temporelle pour un événement (e) et une seconde période temporelle est sélectionnée avec des facteurs d'environnement correspondant étroitement à la première période pour répliquer l'événement (e).

**6.** Procédé de la revendication 5, dans lequel :

une premier stade est un bloc de processus d'observation (242) modélisant une dépendance de données à partir des données mesurées (y) vers le champ de mesurande (x), dans lequel le premier stade modélise une fonction de calibration ; et
un seconde stade est un bloc de réponse d'environnement (241) modélisant une dépendance de données à partir du champ de mesurande (x) vers le champ d'événement (e), dans lequel le second stade modélise la fonction de réponse d'environnement.

**7.** Procédé de la revendication 5 ou de la revendication 6, comprenant l'entraînement d'un modèle à l'aide de données mesurées (y) à partir du capteur, comprenant :

dans la phase de calibration fiable, l'entraînement (112) à la fois du premier stade et du second stade du modèle à l'aide des données mesurées (y) et du champ de mesurande (x), dans lequel le champ de mesurande est déterminé à l'aide de la fonction de réponse de capteur connue (f) ; et
dans la phase de calibration non fiable, le second stade n'est pas entraîné et le premier stade est entraîné (113) uniquement à l'aide des données mesurées (y).

**8.** Procédé de l'une quelconque des revendications 5 à 7, dans lequel, après la phase de calibration non fiable, un bloc de processus d'observation mis à jour du premier stade est utilisé en tant que processus de calibration mis à jour.

**9.** Procédé de l'une quelconque des revendications précédentes, comprenant l'addition (213, 223) d'une représentation (204) de bruit de mesure à la fonction de réponse de capteur connue et à la fonction de réponse de capteur actuelle.

**10.** Procédé de calibration d'un réseau de capteurs, dans lequel chacun des capteurs du réseau présente une position relative connue dans l'environnement donné, le procédé comprenant le procédé de l'une quelconque des revendications 1 à 9 appliqué à chacun des capteurs.

**11.** Système de calibration d'un capteur, dans lequel le capteur est dans un environnement donné, le système comprenant :

un composant de détermination de réponse d'environnement (402) en vue de déterminer une fonction de réponse d'environnement (h) dans une première période temporelle dans une phase de calibration fiable après la calibration du capteur, la fonction de réponse d'environnement (h) étant sur la base d'une fonction de réponse de capteur connue (f), la détermination étant effectuée lors de l'obtention de valeurs mesurées (y) par le capteur d'un mesurande (x) d'un événement (e) dans la première période temporelle, dans lequel la fonction de réponse d'environnement est une réponse spatio-temporelle de facteurs de l'environnement représentant la réponse environnementale opérant sur l'événement pour aboutir au champ de mesurande ;
un composant (403) d'estimation de réponse de capteur actuelle en vue d'estimer, dans une phase de calibration non fiable après la première période temporelle, une fonction de réponse de capteur actuelle sur la base de la fonction de réponse d'environnement (h) déterminée dans la phase de calibration fiable, l'estimation étant effectuée lors de l'obtention de valeurs mesurées (y) par le capteur du champ de mesurande (x) de l'événement (e) dans une seconde période temporelle après la première période temporelle ; et
un composant de sortie (404) en vue d'émettre en sortie une fonction de calibration sur la base d'un inverse de la fonction de réponse de capteur actuelle estimée en vue de mettre à jour la calibration du capteur.

**12.** Système de la revendication 11, dans lequel le composant de détermination de réponse environnementale (402) comprenant :
un composant de mesure de facteur d'environnement (407) en vue de mesurer des facteurs sélectionnés de l'environnement au cours de la première période temporelle au niveau du capteur pour déterminer la fonction de réponse environnementale (h).

**13.** Système de l'une quelconque des revendications 11 à 12, dans lequel le système est un système de calibration aveugle comprenant un réseau d'autocodeur à deux stades (430), chaque stade étant mis en œuvre par un bloc de réseaux neuronaux convolutifs, dans lequel le réseau d'autocodeur est entraîné dans la première période temporelle pour un événement (e) et une seconde période temporelle est sélectionnée avec des facteurs d'environnement correspondant étroitement à la première période pour répliquer l'événement (e).

**14.** Système de la revendication 13, comprenant un composant d'entraînement (430) en vue d'entraîner un modèle à l'aide de données mesurées (y) à partir du capteur, comprenant :

un composant d'entraînement de phase de calibration fiable (431) en vue d'entraîner à la fois le premier stade et du second stade le modèle à l'aide des données mesurées (y) et du champ de mesurande (x), dans lequel le champ de mesurande est déterminé à l'aide de la fonction de réponse de capteur connue (f) ; et
un composant d'entraînement de phase de calibration non fiable (432) en vue d'entraîner le premier stade uniquement à l'aide des données mesurées (y) et en s'abstenant d'entraîner le second stade.

**15.** Programme informatique stocké sur un support lisible par ordinateur et pouvant être chargé dans une mémoire interne d'un ordinateur numérique, comprenant des parties de code logiciel, lorsque ledit programme est exécuté sur un ordinateur, en vue de réaliser les étapes de procédé de l'une quelconque des revendications 1 à 10.

100

```
IN RELIABLE CALIBRATION PHASE,
DETERMINE AN ENVIRONMENT
RESPONSE FUNCTION BASED ON A
KNOWN SENSOR RESPONSE FUNCTION
101
```

```
IN UNRELIABLE CALIBRATION PHASE,
ESTIMATE A SENSOR RESPONSE
FUNCTION BASED ON THE DETERMINED
ENVIRONMENT RESPONSE FUNCTION
102
```

```
OUTPUTTING A CALIBRATION
CORRECTION BASED ON THE ESTIMATED
SENSOR RESPONSE FUNCTION FOR
UPDATING THE CALIBRATION OF THE
SENSOR 103
```

**FIG. 1A**

110

```
PROVIDING A TWO STAGE
AUTOENCODER NETWORK, WITH A FIRST
STAGE OBSERVATION PROCESS BLOCK
FOR MODELING A CALIBRATION
FUNCTION AND A SECOND STAGE
ENVIRONMENT RESPONSE BLOCK FOR
MODELING AN ENVIRONMENT RESPONSE
FUNCTION 111
```

```
IN RELIABLE CALIBRATION PHASE, TRAIN
THE FIRST STAGE AND THE SECOND
STAGE USING THE MEASURED DATA AND
THE MEASURAND DATA 112
```

```
IN UNRELIABLE CALIBRATION PHASE,
ONLY TRAIN THE FIRST STAGE USING
THE MEASURED DATA 113
```

```
OUTPUTTING A CALIBRATION
CORRECTION BASED ON AN UPDATED
OBSERVATION PROCESS BLOCK OF THE
FIRST STAGE 114
```

**FIG. 1B**

FIG. 2A

220

EVENT
(e) 201

ML BLOCK FOR
ENVIRONMENT
RESPONSE (h) 221

MEASURAND
FIELD (x) 203

ML BLOCK FOR
OBSERVATION
PROCESS OF
SENSOR (f) 222

NOISE
REPRESENTATION
(n) 204

ADDITION OF NOISE
REPRESENTATION
223

MEASUREMENT (y)
205

**FIG. 2B**

240

MEASUREMENT (y)
231

ML BLOCK FOR
OBSERVATION
PROCESS OF
SENSOR (f) 242

MEASURAND
FIELD (x) 232

ML BLOCK FOR
ENVIRONMENT
RESPONSE (h) 241

EVENT FIELD (e)
233

ML BLOCK FOR
ENVIRONMENT
RESPONSE (h) 241

MEASURAND
FIELD ($\hat{x}$) 234

ML BLOCK FOR
OBSERVATION
PROCESS OF
SENSOR (f) 242

MEASUREMENT ($\hat{y}$)
235

**FIG. 2C**

310

**Measurand vs sensor-reading (variable *x* vs *y*)**

**FIG. 3A**

320

**Measurand vs sensor-reading (variable *x* vs *y*)**

**FIG. 3B**

330

**FIG. 3C**

340

**FIG. 3D**

350

**FIG. 3E**

360

**FIG. 3F**

CALIBRATION SYSTEM 400

CONTROLLED PERTURBATION
COMPONENT 410

SENSOR INPUT RECEIVING
COMPONENT 401

NOISE REPRESENTING
COMPONENT 407

TWO-STAGE AUTOENCODER NETWORK 420

ENVIRONMENT RESPONSE
DETERMINING COMPONENT
402

ENVIRONMENTAL FACTOR
MEASURING COMPONENT 407

FUNCTION SHAPE
COMPONENT 405

FUNCTION SHAPE
AGGREGATOR
COMPONENT 406

CURRENT SENSOR RESPONSE
ESTIMATING COMPONENT
403

TRAINING COMPONENT 430

RC PHASE TRAINING
COMPONENT 431

UC PHASE TRAINING
COMPONENT 432

OUTPUT COMPONENT
404

**FIG. 4**

**FIG. 5**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10008770 B2
- WO 2020191980 A1

**Non-patent literature cited in the description**

- Blind calibration of sensor networks. **BALZANO L. et al.** Information Processing in Sensor Networks, 2007. IPSN 2007. 6th Int. Symp, 25 April 2007
- **M. G. COX** ; **G. B. ROSSI** ; **P. M. HARRIS** ; **A. FORBES**. A probabilistic approach to the analysis of measurement processes. *Metrologia*, 2008, vol. 45 (5), 493
- **I. GOODFELLOW** ; **Y. BENGIO** ; **A. COURVILLE**. Deep learning. MIT press, 2016
- **K. YAMAMOTO** ; **T. TOGAMI** ; **N. YAMAGUCHI** ; **S. NINOMIYA**. Machine learning-based calibration of low-cost air temperature sensors using environmental data. *Sensors*, 2017, vol. 17 (6), 1290
- **F. CONCAS** ; **J. MINERAUD** ; **E. LAGERSPETZ** ; **S. VARJONEN** ; **X. LIU** ; **K. PUO- LAMA KI** ; **P. NURMI** ; **S. TARKOMA**. Low-cost outdoor air quality monitoring and sensor calibration: A survey and critical analysis. *ACM Transactions on Sensor Networks (TOSN*, 2021, vol. 17 (2), 1-44